# EUROPEAN PATENT APPLICATION

(11) **EP 4 702 971 A1**
(43) Date of publication of application: **04.03.2026**
(21) Application number: 24209548.7
(22) Date of filing: 29.10.2024
(51) Int. Cl.: A61K 9/00, A61K 9/20

(54) **METHOD FOR PREPARATION OF LISDEXAMFETAMINE DIMESYLATE DISPERSIBLE TABLET**

(30) Priority: 03.09.2024 IN 202411066703
(71) Applicant: Athena Pharmaceutiques SAS, 78430 Louveciennes (FR)
(72) Inventor: CHAUDHARI, Mahendra Baliram, 400602 Maharashtra (IN); CHANDWANI, Omprakash Doulatram, 421301 Maharashtra (IN); NEHETE, Nitin Pandharinath, 421201 Maharashtra (IN); CHAUDHARI, Amol Yuvraj, 421202 Maharashtra (IN); SHAHANE, Anita Kunal, 421301 Maharashtra (IN)
(74) Representative: Grünecker Patent- und Rechtsanwälte PartG mbB

(57) **Abstract**

The present disclosure relates to a method for preparation of a lisdexamfetamine dimesylate dispersible tablet. The method for preparation comprises: dry mixing lisdexamfetamine dimesylate, a diluent, a binder and an anti-static agent to form a dry mix blend; intermediate granular blending of a filler, a cushioning agent, a disintegrating agent, and a sweetener to form an intermediate granular blend; adding and blending the dry mix blend and the intermediate granular blend to form an intermediate drug blend; lubricating the intermediate drug blend with a lubricant to form ready to compress drug granules; and compressing the ready to compress drug granules to form a lisdexamfetamine dimesylate dispersible tablet.

## Description

### FIELD OF THE PRESENT DISCLOSURE

The present disclosure generally relates to a method for preparation of a dispersible tablet. In particular, the present disclosure relates to a method for preparation of a lisdexamfetamine dimesylate dispersible tablet.

### BACKGROUND

Lisdexamfetamine dimesylate, the dimesylate salt of L-Lysyl-d-amfetamine with molecular formula C₁₇H₃₃N₃O₇S₂ is also known by a chemical name "(2S)-2,6-diamino-N-[(1S)-1-methyl-2-phenylethyl]-hexanamide, dimethanesulfonate". IUPAC name of the Lisdexamphetamine dimesylate is (2S)-2,6-diamino-N-[(2S)-1-phenylpropan-2-yl] hexanamide; methanesulfonic acid.

Chemical structure of Lisdexamfetamine dimesylate is represented below:

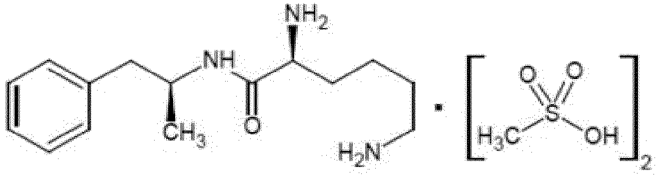

Lisdexamfetamine dimesylate generally indicates the treatment of Attention Deficit Hyperactivity Disorder (ADHD) and moderate to severe Binge Eating Disorder (BED) in adults.

Lisdexamfetamine dimesylate is highly deliquescent material by its nature, which on being exposed to the moisture by any means absorbs the moisture and does not remain in solid form.

Lisdexamfetamine dimesylate is present in the market in the form of capsules, and chewable tablets. Multitude of patient's group such as geriatrics, patients with mental retardness, uncooperative patients, patients with nausea, and patients with particular or customised diet etc., face difficulty in swallowing lisdexamfetamine dimesylate in the available capsules and chewable tablets dosage forms.

During the last decade there was a need for an upcoming generation of pharmaceutical products and medicaments in multiple dosage amounts which can be consumed with ease, which gets rapidly dissolved and have the characteristics of dissolving or melting or disintegrating in only a few seconds and providing desired therapeutic outcome. The need further includes a suitable type of method for preparation of formulation enhancing the disintegration; dissolution, and free flowability of the components in the granule of the dosage form by physico-chemical parameters adopted during the method.

Accordingly, there is a need for lisdexamfetamine dimesylate composition and dosage form that addresses the deliquescence problem of lisdexamfetamine dimesylate. It is therefore highly desirable to develop a lisdexamfetamine dimesylate dosage form which disintegrates fast and permits rapid release of the drug from the granules and allows rapid absorption in the body after administration. Further, it is desirable to develop a lisdexamfetamine dimesylate dispersible tablet that is stable, palatable, and disintegrates fast. Moreover, there is a need for a method in which the process time and production cost for preparing lisdexamfetamine dimesylate composition is low. Furthermore, there is a need for a manufacturing process with controlled humidity to minimize exposure of the deliquescent material to moisture during the production process.

### SUMMARY

To achieve the foregoing and other objects and needs, the present disclosure provides a method for preparation of lisdexamfetamine dimesylate dispersible tablet and the lisdexamfetamine dimesylate dispersible tablet prepared thereby. The lisdexamfetamine dimesylate dispersible tablet developed by the inventor is a dispersible tablet in multiple dosage amount which can be consumed with ease, which gets rapidly dissolved and have the characteristics of dissolving or melting or disintegrating in only a few seconds and providing desired therapeutic outcome. The present disclosure provides a method for preparation of a dispersible tablet which enhances the disintegration; dissolution, and free flowability of the components of the granule in the dosage form by its physico-chemical parameters adopted during the method.

The present disclosure provides a synergistic combination of the API (active pharmaceutical ingredient) with the excipients in specific ratio to address the deliquescent nature of lisdexamfetamine dimesylate. The synergistic combination enhances the disintegration time of the dispersible tablet. The present disclosure further imparts improved palatability to the tablet formulation of lisdexamfetamine dimesylate. The present disclosure provides a humidity-controlled method of preparation wherein the tablet formulation is manufactured in an environment with controlled humidity to minimize exposure of the deliquescent material to moisture during the production process. Particularly, the manufacturing process employs direct compression techniques during the manufacturing process in controlled humidity levels of NMT 40% to reduce exposure to moisture.

The present disclosure provides a method for preparation of lisdexamfetamine dimesylate composition for dispersible tablets which overcomes the drawbacks of solid compositions like capsules and chewable tablets. The synergistic combination of the API with the excipients in specific ratio is formulated to address the deliquescent nature of lisdexamfetamine dimesylate and provide a stable lisdexamfetamine dimesylate dispersible composition. The dissolution of the lisdexamfetamine dispersible tablet composition is not less than 75% in 15 minutes.

In an embodiment, the present disclosure relates a method for preparation of a lisdexamfetamine dimesylate dispersible tablet. The method comprises dry mixing lisdexamfetamine dimesylate, a diluent, a binder and an anti-static agent to form a dry mix blend. Thereafter, intermediate granular blending of a filler, a cushioning agent, a disintegrating agent, and a sweetener to form an intermediate granular blend. Thereafter, adding and blending the dry mix blend and the intermediate granular blend to form an intermediate drug blend. Thereafter, lubricating the intermediate drug blend with a lubricant to form ready to compress drug granules; and finally, compressing the ready to compress drug granules to form a lisdexamfetamine dimesylate dispersible tablet.

In one or more embodiments, the lisdexamfetamine dimesylate is present in an amount of about 8 to about 12 percent by weight.

In one or more embodiments, in the dry mixing step, the diluent is mannitol granular 200SD, and the diluent is present in an amount of about 18 to about 23 percent by weight. The binder is partially pregelatinized maize starch, and the binder is present in an amount of about 10 to about 20 percent by weight. The anti-static agent is colloidal anhydrous silica, and the anti-static agent is present in an amount of about 1 to about 2 percent by weight.

In one or more embodiments, in the intermediate granular blending step, the filler is mannitol granular 200SD, and the diluent is present in an amount of about 28 to about 34 percent by weight. The cushioning agent is microcrystalline cellulose, and the cushioning agent is present in an amount of about 8 to about 12 percent by weight. The disintegrating agent is crospovidone, and the disintegrating agent is present in an amount of about 6 to about 10 percent by weight. The sweetener is sucralose, and the sweetener is about 1 to about 4 percent by weight.

In one or more embodiments, in the lubrication step, the lubricant is magnesium stearate, and the lubricant is present in an amount of about 1 to about 2 percent by weight.

In one or more embodiments, the dosage amount of the lisdexamfetamine dimesylate dispersible tablet is 10 mg per tablet; 20 mg per tablet; 30 mg per tablet; 40 mg per tablet; 50 mg per tablet; 60 mg per tablet; and 70 mg per tablet.

In one or more embodiments, the dissolution is not less than 75% in 15 minutes.

In one or more embodiments, the friability is not more than 1.5%, the disintegration time is less than 3 minutes at 15°C to 25°C (without disc), and the hardness is from about 30 N to about 70 N.

In one or more embodiments, the step of compressing employs direct compression technique in controlled humidity level of NMT 40% to reduce exposure to moisture.

In one or more embodiments, the method further comprises packaging of lisdexamfetamine dimesylate dispersible tablets in moisture resistant ALU peel off blister package; and/or moisture resistant ALU-ALU blister package.

### BRIEF DESCRIPTION OF THE FIGURES

The advantages and features of the present disclosure will become better understood with reference to the following detailed description and claims taken in conjunction with the accompanying drawing, in which:
FIG. 1 illustrates a method flow of a lisdexamfetamine dimesylate dispersible tablet, in accordance with an exemplary embodiment of the present disclosure.
FIG. 2 illustrates method details of dry mix blend, in accordance with an exemplary embodiment of the present disclosure.
FIG. 3 illustrates method details of intermediate granular blend, in accordance with an exemplary embodiment of the present disclosure.
FIG. 4 illustrates method details of preparation of intermediate drug blend, in accordance with an exemplary embodiment of the present disclosure.
FIG. 5 illustrates method details of preparation of ready to compress drug granules, in accordance with an exemplary embodiment of the present disclosure.
FIG. 6 illustrates method details of lisdexamfetamine dimesylate dispersible tablet, in accordance with an exemplary embodiment of the present disclosure.

### DETAILED DESCRIPTION

The exemplary embodiments described herein detail for illustrative purposes are subject to many variations in structure and design. It should be emphasized, however, that the present disclosure is not limited to the method for preparation of lisdexamfetamine dimesylate dispersible tablet, as shown and described. It is understood that various omissions and substitutions of equivalents are contemplated as circumstances may suggest or render expedient, but these are intended to cover the application or implementation without departing from the spirit or scope of the claims of the present disclosure. Also, it is to be understood that the phraseology and terminology used herein is for the purpose of description and should not be regarded as limiting.

The use of terms "including," "comprising," or "having" and variations thereof herein is meant to encompass the items listed thereafter and equivalents thereof as well as additional items.

Further, the terms, "a" and "an" herein do not denote a limitation of quantity, but rather denote the presence of at least one of the referenced items.

As used herein, the term "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the particular term.

The present disclosure provides a method for preparation of lisdexamfetamine dimesylate dispersible tablet and the lisdexamfetamine dimesylate dispersible tablet prepared thereby. The lisdexamfetamine dimesylate dispersible tablet developed by the inventor is a dispersible tablet in multiple dosage amount which can be consumed with ease, which gets rapidly dissolved and have the characteristics of dissolving or melting or disintegrating in only a few seconds and providing desired therapeutic outcome. The present disclosure provides a method for preparation of dispersible tablet which enhances the disintegration; dissolution, and free flowability of the components of the granule in the dosage form by its physico-chemical parameters adopted during the method.

The lisdexamfetamine dimesylate dispersible tablet addresses the deliquescence problem of lisdexamfetamine dimesylate. The lisdexamfetamine dimesylate dispersible tablet disintegrates fast and permits rapid release of the drug from the granules and allows rapid absorption in the body after administration. Further, the lisdexamfetamine dimesylate dispersible tablet is stable, palatable, and disintegrates fast. Moreover, the method for preparation of lisdexamfetamine dimesylate tablet involves lesser process time and production cost. Furthermore, the method has controlled humidity to minimize exposure of the deliquescent material to moisture during the production of dispersible tablet.

The lisdexamfetamine dimesylate dispersible tablets are quickly disintegrable or soluble and the tablet in dispersible form on consumption lets the drug be absorbed in the gastrointestinal regions.

The present disclosure provides a synergistic combination of the API with the excipients in specific ratio to address the deliquescent nature of lisdexamfetamine dimesylate. The synergistic combination enhances the disintegration time of the dispersible tablet. The present disclosure further imparts improved palatability to the tablet formulation of lisdexamfetamine dimesylate. The present disclosure provides a humidity-controlled method of preparation wherein the tablet formulation is manufactured in an environment with controlled humidity to minimize exposure of the deliquescent material to moisture during the production process. Particularly, the manufacturing process employs direct compression techniques during the manufacturing process in controlled humidity level of NMT 40% to reduce exposure to moisture.

The present disclosure provides a method for preparation of lisdexamfetamine dimesylate composition for dispersible tablet which overcomes the drawbacks of solid compositions like capsules and chewable tablets. The synergistic combination of the API with the excipients in specific ratio is formulated to address the deliquescent nature of lisdexamfetamine dimesylate and provide a stable lisdexamfetamine dimesylate dispersible composition. The dissolution of the lisdexamfetamine dispersible tablet composition is not less than 75% in 15 minutes.

In one embodiment, a method for preparation of a lisdexamfetamine dimesylate dispersible tablet is disclosed. Herein, the "method for preparation of a lisdexamfetamine dimesylate dispersible tablet" and "method" have been interchangeably used hereinafter without any limitations. A method for preparation of a lisdexamfetamine dimesylate dispersible tablet, the method comprising dry mixing lisdexamfetamine dimesylate, a diluent, a binder and an anti-static agent to form a dry mix blend. Intermediate granular blending of a filler, a cushioning agent, a disintegrating agent, and a sweetener to form an intermediate granular blend. Adding and blending the dry mix blend and the intermediate granular blend to form an intermediate drug blend. Lubricating the intermediate drug blend with a lubricant to form ready to compress drug granules. Finally, compressing the ready to compress drug granules to form a lisdexamfetamine dimesylate dispersible tablet. Herein, the particle size for a drug granule forming the lisdexamfetamine dimesylate dispersible tablet is not more than 400 microns.

The method for preparation of a lisdexamfetamine dimesylate dispersible tablet majorly comprises the steps of dry mixing; intermediate blending; lubrication; and compression. Specifically, resulting in preparation of dry mix blend; preparation of intermediate granular blend; preparation of intermediate drug blend; preparation of ready to compress drug granules; and preparation of lisdexamfetamine dimesylate dispersible tablet.

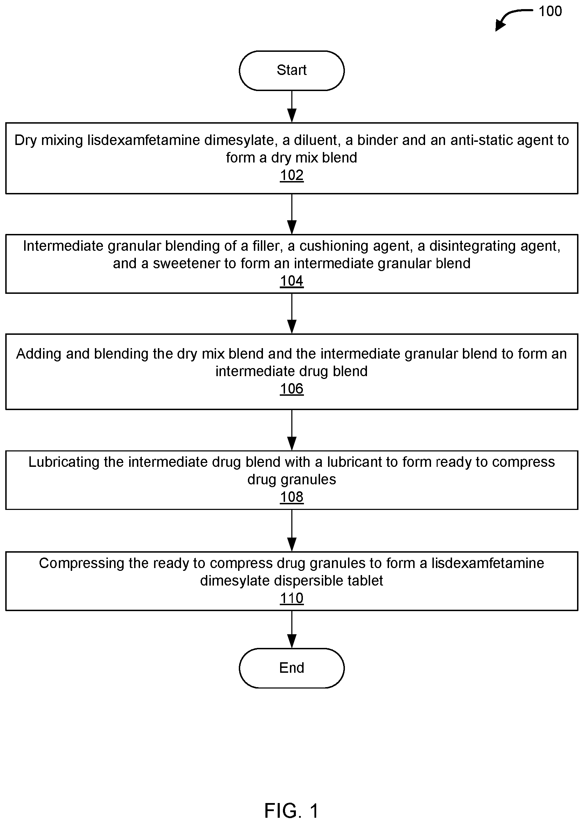

Referring to FIG. 1, the method 100 relates to preparation of a lisdexamfetamine dimesylate dispersible tablet. At step 102, the method 100 comprises dry mixing lisdexamfetamine dimesylate, a diluent, a binder and an anti-static agent to form a dry mix blend. At step 104, the method 100 comprises intermediate granular blending of a filler, a cushioning agent, a disintegrating agent, and a sweetener to form an intermediate granular blend. At step 106, the method 100 comprises adding and blending the dry mix blend and the intermediate granular blend to form an intermediate drug blend. At step 108, the method 100 comprises lubricating the intermediate drug blend with a lubricant to form ready to compress drug granules. At step 110, the method 100 comprises compressing the ready to compress drug granules to form a lisdexamfetamine dimesylate dispersible tablet. Thereafter, the lisdexamfetamine dimesylate dispersible tablets are packaged in moisture resistant ALU peel off blister package / ALU-ALU blister package. The moisture resistant ALU peel off blister package /ALU-ALU blister package contributes to the humidity-controlled method of preparation and packaging.

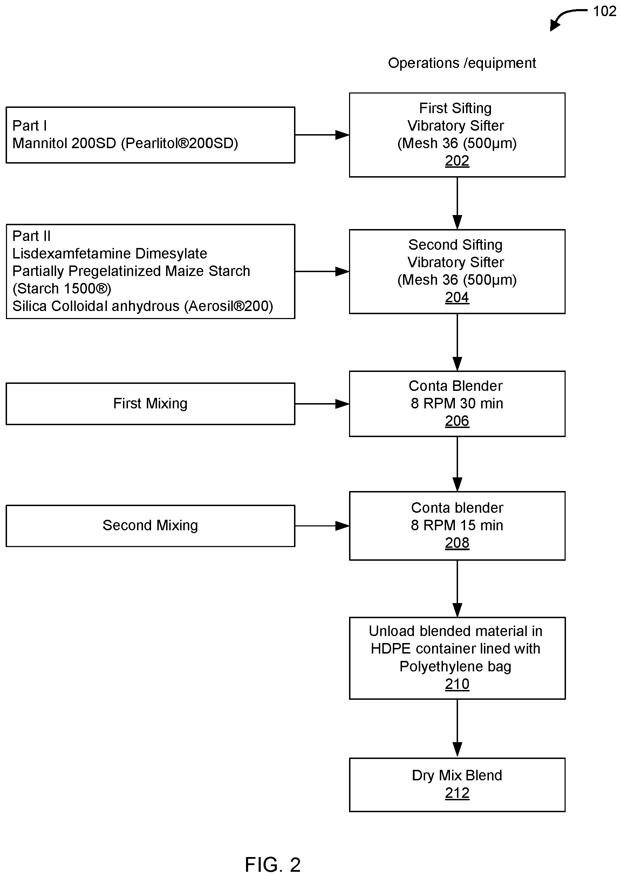

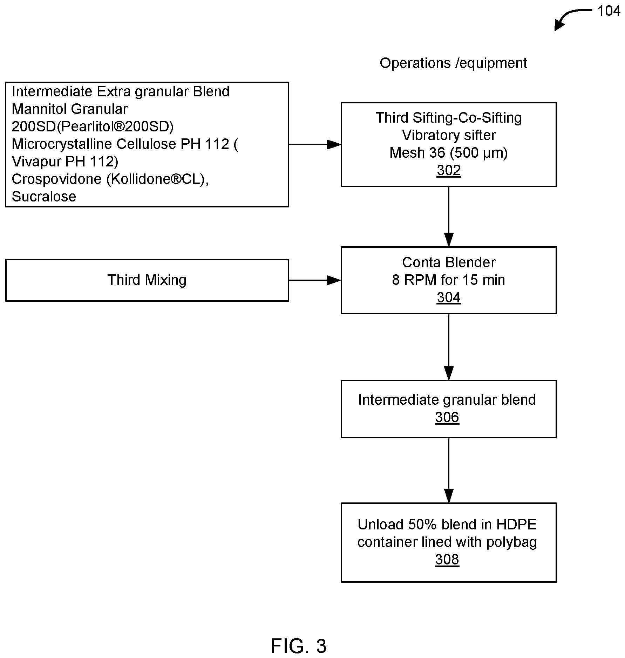

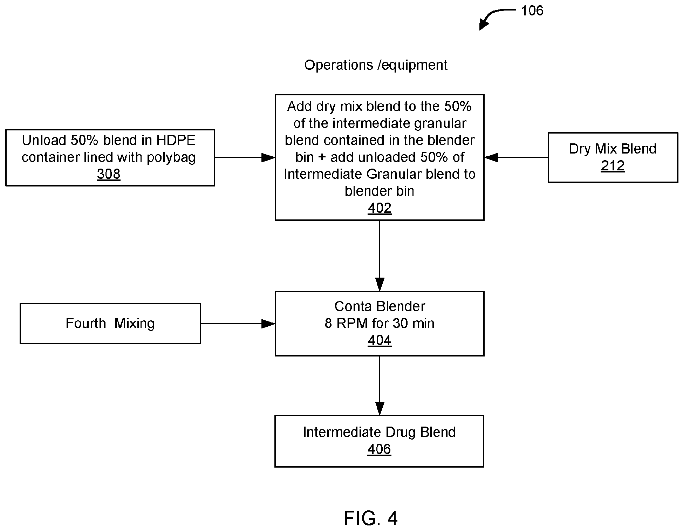

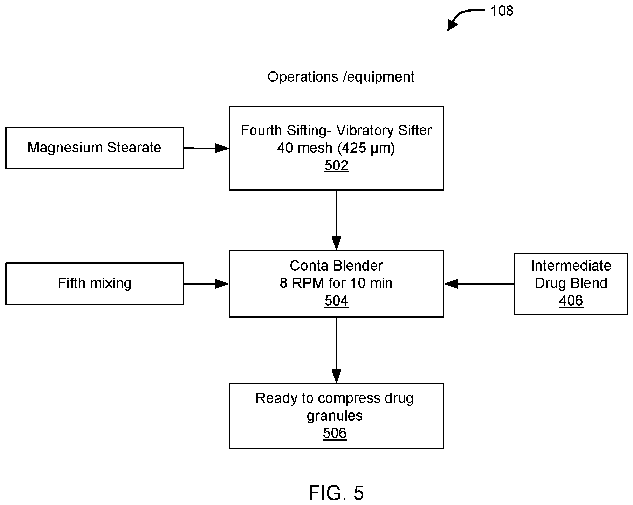

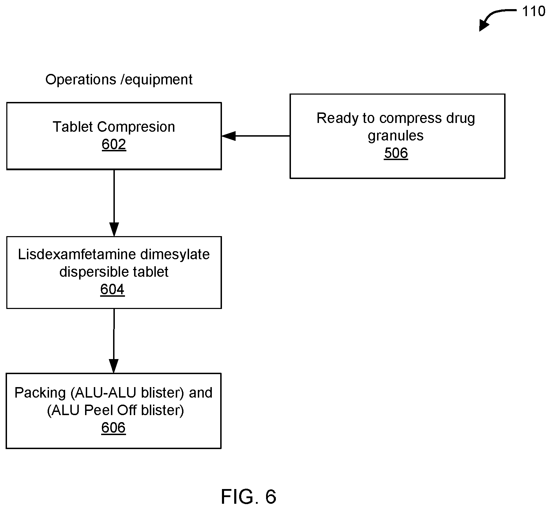

The method step 102 of FIG. 1 is described in detail in FIG. 2. The method step 104 of FIG. 1 is described in detail in FIG. 3. The method step 106 of FIG. 1 is described in detail in FIG. 4. The method step 108 of FIG. 1 is described in detail in FIG. 5. The method step 110 of FIG. 1 is described in detail in FIG. 6.

Again, referring to FIG. 1, at step 102, the method 100 involves dry mixing lisdexamfetamine dimesylate, a diluent, a binder and an anti-static agent to form a dry mix blend. The method of preparation step 102 of FIG. 1 is described in detail in FIG. 2.

Referring to FIG. 2, at step 202, in part I, the diluent, which is mannitol granular 200SD is sifted through 36 mesh of a vibratory sifter to obtain agglomerate free mass of the diluent. In an example, the mannitol granular 200SD is Pearlitol^{®}200SD.

Referring to FIG. 2, at step 204, in part II, the lisdexamfetamine dimesylate; the binder, which is partially pregelatinized maize starch; the anti-static agent, which is colloidal anhydrous silica are co-sifted through 36 mesh of the vibratory sifter to obtain agglomerate free mass of lisdexamfetamine dimesylate, the binder and the anti-static agent. In an example, the partially pregelatinized maize starch is Starch1500^{®}. In an example, colloidal anhydrous silica is Aerosil^{®} 200.

At step 206, as a first level of mixing, the agglomerate free mass of lisdexamfetamine dimesylate, the binder and the anti-static agent obtained at step 204 is blended in a conta blender at 8 RPM for 30 minutes to form blended mixture of lisdexamfetamine dimesylate, the binder and the anti-static agent.

At step 208, as a second level of mixing, the blended mixture obtained at step 206 is further blended in the conta blender at 8 RPM for 15 minutes to obtain blended material.

At step 210, the blended material obtained at step 208 is unloaded in HDPE container lined with polyethylene bag and added with agglomerate free mass of the diluent obtained at step 202 to form dry mix blend at step 212.

Again, referring to FIG. 1, at step 104, the next step in the method 100 involves intermediate granular blending of a filler, a cushioning agent, a disintegrating agent, and a sweetener to form an intermediate granular blend. The method of preparation step 104 of FIG. 1 is described in detail in FIG. 3.

Referring to FIG. 3, at step 302, the filler, which is mannitol granular 200SD; the cushioning agent, which is microcrystalline cellulose; the disintegrating agent, which is crospovidone; the sweetener, which is sucralose are co-sifted through 36 mesh of the vibratory sifter to obtain agglomerate free mass of the sifted filler, cushioning agent, disintegrating agent, and sweetener. In an example, the mannitol granular 200SD is Pearlitol^{®}200SD. In an example, microcrystalline cellulose is Vivapur^{®} 112. In an example, crospovidone is KollidoneCL.

At step 304, the agglomerate free mass of the sifted filler, cushioning agent, disintegrating agent, and sweetener obtained at step 302 is blended in the conta blender at 8 RPM for 15 minutes. Thereafter, at step 306, intermediate granular blend is obtained. Further, the intermediate granular blend obtained at step 306 is divided into two parts as first half of the intermediate granular blend and second half of the intermediate granular blend.

At step 308, the first half of the intermediate granular blend is unloaded in the HDPE container lined with polybag, hereinafter alternatively termed as "unloaded 50% of the intermediate granular blend" and the second half of the intermediate granular blend is left in the bin of the conta blender, hereinafter alternatively termed as "50% of the intermediate granular blend contained in the blender bin".

Again, referring to FIG. 1, at step 106, the next step in the method 100 involves adding and blending the dry mix blend obtained at step 212 and the intermediate granular blend obtained at step 306 to form an intermediate drug blend. The method of preparation step 106 of FIG. 1 is described in detail in FIG. 4.

Referring to FIG. 4, at step 402, the dry mix blend obtained at step 212 in the FIG. 2 is added to the "50% of the intermediate granular blend contained in the blender bin" (second half) and further the "unloaded 50% of the intermediate granular blend" (first half) divided at step 308 is added to the blender bin already containing the second half of the intermediate granular blend and dry mix blend. Post addition of the second half of the intermediate granular blend; dry mix blend; and the first half of the intermediate granular blend sequentially to the bin of the conta blender, at step 404, the mixture is blended at 8 RPM for 30 minutes to obtain intermediate drug blend at step 406.

Again, referring to FIG. 1, at step 108, the next step in the method 100 involves lubricating the intermediate drug blend with a lubricant to form ready to compress drug granules. The method of preparation step 108 of FIG. 1 is described in detail in FIG. 5.

Referring to FIG. 5, at step 502, the lubricant, which is magnesium stearate, is sifted through 40 mesh of the vibratory sifter to obtain agglomerate free mass of the sifted lubricant. Further, at step 504, the sifted lubricant obtained at step 502 is added to the intermediate drug blend obtained at step 406 in FIG. 4 and blended in the conta blender at 8 RPM for 10 minutes to obtain ready to compress drug granules at step 506.

Again, referring to FIG. 1, at step 110, the next step in the method 100 involves compressing of ready to compress drug granules to form a lisdexamfetamine dimesylate dispersible tablet. The method of preparation step 110 of FIG. 1 is described in detail in FIG. 6.

Referring to FIG. 6, at step 602, the ready to compress drug granules obtained at step 506 in FIG. 5 are compressed by employing direct compression technique involving controlled humidity level of NMT 40% to reduce exposure to moisture to form a lisdexamfetamine dimesylate dispersible tablet.

In one or more embodiments, the lisdexamfetamine dimesylate is present in an amount of about 8 to about 12 percent by weight.

As used herein, lisdexamfetamine dimesylate is the active substance. Lisdexamfetamine dimesylate, the dimesylate salt of L-Lysyl-d-amfetamine with molecular formula C₁₇H₃₃N₃O₇S₂ is also known by a chemical name "(2S)-2,6-diamino-N-[(1S)-1-methyl-2-phenylethyl]-hexanamide, dimethanesulfonate". IUPAC name of the Lisdexamphetamine dimesylate is (2S)-2,6-diamino-N-[(2S)-1-phenylpropan-2-yl] hexanamide; methanesulfonic acid. As used in the present disclosure, the particle size of the lisdexamfetamine dimesylate is not more than 100 microns.

Structural formula of lisdexamfetamine dimesylate shows that the molecule of lisdexamfetamine dimesylate contains two chiral centres. Thus, there are four possible enantiomers of this compound: (2S)-2,6-diamino-N-[(2S)-1-phenylpropan-2-yl]hexanamide; (2R)-2,6-diamino-N-[(2S)-1-phenylpropan-2-yl]hexanamide; (2S)-2,6-diamino-N-[(2R)-1-phenylpropan-2-yl]hexanamide; and (2R)-2,6-diamino-N-[(2R)-1-phenylpropan-2-yl]hexanamide.

Lisdexamfetamine dimesylate is a white or almost white crystalline powder which is therapeutically categorized as a CNS stimulant drug also called amfetamines. Lisdexamfetamine dimesylate is freely soluble in methanol; soluble in ethanol; very slightly soluble in acetone, chloroform and toluene; and practically insoluble in tetrahydrofuran.

Characteristically, lisdexamfetamine dimesylate is hygroscopic in nature and generally indicates the treatment of Attention Deficit Hyperactivity Disorder (ADHD), and moderate to severe binge eating disorder (BED) in adults.

In one or more embodiments, in the dry mixing step, the diluent is mannitol granular 200SD, and the diluent is present in an amount of about 18 to about 23 percent by weight.

As used herein, the mannitol granular 200SD is used in preparation of the lisdexamfetamine dimesylate dispersible tablet. Mannitol granular 200SD is used to adjust the average weight of lisdexamfetamine dimesylate dispersible tablets. Mannitol granular 200SD also enhances the flow properties and mouth feel of lisdexamfetamine dimesylate dispersible tablet formulation. Mannitol granular 200SD is used in the present disclosure in two parts, first as a diluent and second as a filler, to enhance the uniformity and distribution of drugs in the formulation. In an example, the mannitol granular 200SD is Pearlitol^{®}200SD.

In one or more embodiments, in the dry mixing step, the binder is partially pregelatinized maize starch, and the binder is present in an amount of about 10 to about 20 percent by weight.

As used herein, the partially pregelatinized maize starch is used in preparation of the lisdexamfetamine dimesylate dispersible tablet. Lisdexamfetamine dimesylate as an active substance is a deliquescent molecule, by that virtue, on keeping it at room temperature conditions it converts in liquid form. Therefore, in order to keep the active lisdexamfetamine dimesylate in its solid form, swellable partially pregelatinized maize starch is used in this lisdexamfetamine dimesylate dispersible tablet formulation. Partially pregelatinized maize starch improves the flow properties of powder guaranteeing uniformity. In contact with aqueous dissolution medium the pregelatinized maize starch swells and triggers the disintegration. It significantly decreases the adhesion of powders. In an example, the partially pregelatinized maize starch is Starch1500^{®}.

In one or more embodiments, in the dry mixing step, the anti-static agent is colloidal anhydrous silica, and the anti-static agent is present in an amount of about 1 to about 2 percent by weight.

As used herein, colloidal anhydrous silica is used in preparation of the lisdexamfetamine dimesylate dispersible tablet. Colloidal anhydrous silica is an adsorbent, anticaking agent, and glidant. It enhances the flow of powder. In an example, colloidal anhydrous silica is Aerosil^{®} 200.

In one or more embodiments, in the intermediate granular blending step, the filler is mannitol granular 200SD, and the filler is present in an amount of about 28 to about 34 percent by weight.

As used herein, the mannitol granular 200SD is used in preparation of the lisdexamfetamine dimesylate dispersible tablet as a filler at the intermediate granular blending step. Mannitol granular 200SD is used to adjust the average weight of lisdexamfetamine dimesylate dispersible tablets. Mannitol granular 200SD also enhances the flow properties and mouth feel of lisdexamfetamine dimesylate dispersible tablet formulation. Mannitol granular 200SD is used in the present disclosure in two parts, first as a diluent and second as a filler, to enhance the uniformity and distribution of drugs in the formulation. In an example, the mannitol granular 200SD is Pearlitol^{®}200SD.

In one or more embodiments, in the intermediate granular blending step, the cushioning agent is microcrystalline cellulose, and the cushioning agent is present in an amount of about 8 to about 12 percent by weight.

As used herein, the microcrystalline cellulose is used in preparation of the lisdexamfetamine dimesylate dispersible tablet. Microcrystalline cellulose is crystalline in nature. Microcrystalline cellulose enhances the flow of powder and helps in tablet compression to give desired hardness. It also acts as a cushioning agent. Due to the plastic nature of microcrystalline cellulose, it is often used as an excipient for both tablet compression and pelletizing processes. Microcrystalline cellulose as a cushioning agent fills the space between the particles and dampens the effect of the compressive force occurring through the tableting process. In an example, microcrystalline cellulose is Vivapur^{®} 112.

In one or more embodiments, in the intermediate granular blending step, the disintegrating agent is crospovidone, and the disintegrating agent is present in an amount of about 6 to about 10 percent by weight.

As used herein, crospovidone is used in preparation of the lisdexamfetamine dimesylate dispersible tablet. Crospovidone is a tablet disintegrant and dissolution agent. Crospovidone rapidly exhibits high capillary activity and pronounced hydration capacity with little tendency to form gels. In an example, crospovidone is Kollidone^{®}CL.

In one or more embodiments, in the intermediate granular blending step, the sweetener is sucralose, and the sweetener is present in an amount of about 1 to about 4 percent by weight.

As used herein, sucralose is used in preparation of the lisdexamfetamine dimesylate dispersible tablet. Sucralose is white to off-white colored, free flowing, crystalline powder which has sweetening power approximately 300-1000 times that of sucrose and has no after taste.

In one or more embodiments, in the lubrication step, the lubricant is magnesium stearate, and the lubricant is present in an amount of about 1 to about 2 percent by weight.

As used herein, magnesium stearate is used in preparation of the lisdexamfetamine dimesylate dispersible tablet. Magnesium stearate is an impalpable powder with low bulk density. It is very fine and greasy in texture, light in weight, white in color, precipitated or milled in nature. Functionally, it is used as a lubricant in capsule and tablet manufacturing. Magnesium stearate powder, because of its characteristic greasiness adheres readily to the surface of tablet to ensure tablet is cleanly ejected without cracking or breakage in the pharmaceutical industry.

In one or more embodiments, the dosage amount of the lisdexamfetamine dimesylate dispersible tablet is 10 mg per tablet; 20 mg per tablet; 30 mg per tablet; 40 mg per tablet; 50 mg per tablet; 60 mg per tablet; and 70 mg per tablet.

In one or more embodiments, the dissolution is not less than 75% in 15 minutes.

In one or more embodiments, the friability is not more than 1.5%, the disintegration time is less than 3 minutes at 15°C to 25°C (without disc), and the hardness is from about 30 N to about 70 N.

In one or more embodiments, the step of compressing employs direct compression technique in controlled humidity level of NMT 40% to reduce exposure to moisture.

Again, referring to FIG. 6, after compression of lisdexamfetamine dimesylate dispersible tablet at step 602, the lisdexamfetamine dimesylate dispersible tablet is obtained at step 604. The lisdexamfetamine dimesylate dispersible tablet obtained at step 604 is stored in black coloured double-lined polybags in HDPE drums containing silica gel bags in pouch as desiccant. Drums are stored at a temperature less than 25°C in the quarantine area before primary packaging. Herein, the compression yield is more than 95%.

In one or more embodiments, the method for preparation further comprises packaging of lisdexamfetamine dimesylate dispersible tablets in moisture resistant ALU peel off blister package; and/or moisture resistant ALU-ALU blister package.

Again, referring to FIG. 6, at step 606, the lisdexamfetamine dimesylate dispersible tablet obtained at step 604 are packaged. In one embodiment, the compressed lisdexamfetamine dimesylate dispersible tablets are packed in moisture resistant ALU peel off blister package. In another embodiment, the compressed lisdexamfetamine dimesylate dispersible tablets are packed in moisture resistant ALU-ALU blister package.

The dispersible tablet of lisdexamfetamine dimesylate rapidly disperses in water to produce a homogeneous dispersion that ensures uniformity of dose and desired therapeutic outcome.

Dispersible tablets as defined in Ph. Eur. are uncoated or film coated tablets intended to be dispersed in water before administration giving a homogeneous dispersion. Typically, a dispersible tablet is dispersed in about 5-15 ml of water and the resulting dispersion is administered to the patient. Dispersible tablets are required to disintegrate within 3 minutes in water at 15-25° C. Also, the dispersion produced from a dispersible tablet should pass through a sieve screen with a nominal mesh aperture of 710 microns. The dosage form provides advantages of both tablets and liquid formulations. These are convenient to carry, easy to manufacture and more stable.

In TABLE 1, the weight percentage of API and excipients at different steps of the method for preparation is depicted.

**TABLE 1**

| **DRY MIXING** | | |
|---|---|---|
| **Ingredients** | **Function** | **Relative Qty. (% w/w)** |
| Lisdexamfetamine dimesylate | Active | 10.0 |
| Mannitol Granular 200SD (Pearlitol^{®} 200SD) | Diluent | 20.75 |
| Partially pregelatinized Maize Starch (Starch1500^{®}) | Binder | 15.0 |
| Colloidal Anhydrous Silica (Aerosil^{®}200) | Anti-static Agent | 1.50 |

| **INTERMEDIATE BLENDING** | | |
|---|---|---|
| Mannitol Granular 200SD (Pearlitol^{®} 200SD) | Filler | 31.0 |
| Microcrystalline Cellulose (VIVAPUR^{®} 112) | Cushioning agent | 10.0 |
| Crospovidone (Kollidone^{®}CL) | Disintegrating Agent | 8.0 |
| Sucralose | Sweetener | 2.50 |

| **LUBRICATION** | | |
|---|---|---|
| Magnesium Stearate | Lubricant | 1.25 |
| Total | | 100.0 |

The method for preparation of lisdexamfetamine dimesylate in an alternative embodiment can be used as a method for preparation of lisdexamfetamine dimesylate orodispersible tablet or ODT tablet.

In TABLE 2, the physical characteristics (in mg/tablet) for 10, 20, 30, 40, 50, 60, and 70 mg/tablets of lisdexamfetamine dimesylate dispersible tablets are depicted.

**TABLE 2**

| **Sr. No** | **Parameters** | **Lisdexamfetamine Dimesylate DT** | | | | | | |
|---|---|---|---|---|---|---|---|---|
| | | **10 mg** | **20 mg** | **30 mg** | **40 mg** | **50 mg** | **60 mg** | **70 mg** |
| **1.** | **Appearance** | White to off white, round, flat beveled edge tablet plain on both surfaces | White to off white, round, flat beveled edge tablet plain on both surfaces | White to off white, round, flat beveled edge tablet plain on both surfaces | White to off white, round, flat beveled edge tablet plain on both surfaces | White to off white, round, flat beveled edge tablet plain on both surfaces | White to off white, round, flat beveled edge tablet plain on both surfaces | White to off white, round, flat beveled edge tablet plain on both surfaces |
| **2.** | **Average Weight of Tablet** | 100.0 mg | 200.0 mg | 300.0 mg | 400.0 mg | 500.0 mg | 600.0 mg | 700.0 mg |
| **3.** | **Weight of 20 Tablets** | 2.0 g | 4.0 g | 6.0 g | 8.0 g | 100 g | 12.0 g | 14.0 g |
| **4.** | **Thickness** | 2.2 mm | 3.6 mm | 3.50 mm | 3.80 mm | 4.0 mm | 4.1 mm | 4.0 mm |
| **5.** | **Diameter** | 7 mm | 8 mm | 10 mm | 11 mm | 12 mm | 13 mm | 14 mm |
| **6.** | **Resistance to crushing strength of tablet / Hardness** | 30 N | 35N | 40 N | 50 N | 50 N | 60 N | 70 N |
| **7.** | **Disintegration time** | NMT 3.0 minutes at 15°C to 25°C (without disc) | | | | | | |
| **8.** | **Friability** | NMT 1.5% | | | | | | |
| **9.** | **Fineness of Dispersion (sieve screen with a nominal mesh aperture of 710 µm)** | Complies | Complies | Complies | Complies | Complies | Complies | Complies |

The lisdexamfetamine dimesylate dispersible tablet and process for preparation thereof is further illustrated by the following non-limiting examples. A person skilled in the art would recognize that the specific examples are intended to illustrate, not limit, the scope of the present disclosure.

### EXAMPLE 1

In Example 1, the method for preparation of lisdexamfetamine dimesylate dispersible tablet is described. Particularly, the method for preparation is described for preparing lisdexamfetamine dimesylate dispersible tablet having strength of 10 mg per tablet.

In TABLE 3, the amount of API and excipients (in mg/tablet) for Lisdexamfetamine Dimesylate (10 mg) dispersible tablets are depicted.

**TABLE 3**

| **DRY MIXING** | |
|---|---|
| **Ingredients** | **Relative Qty. (in mg/tablet)** |
| Lisdexamfetamine dimesylate | 10.0 mg |
| Mannitol Granular 200SD (Pearlitol^{®}200SD) | 20.75 mg |
| Partially pregelatinized Maize Starch (Starch1500^{®}) | 15 mg |
| Colloidal Anhydrous Silica (Aerosil^{®}200) | 1.50 |

| **INTERMEDIATE BLENDING** | |
|---|---|
| Mannitol Granular 200SD (Pearlitol^{®}200SD) | 31 mg |
| Microcrystalline Cellulose (VIVAPUR^{®} 112) | 10 mg |
| Crospovidone (Kollidone^{®}CL) | 8 mg |
| Sucralose | 2.50 mg |

| **LUBRICATION** | |
|---|---|
| Magnesium Stearate | 1.25 mg |
| Total | 100.0 mg |

The method is initiated by dry mixing lisdexamfetamine dimesylate, a diluent, a binder and an anti-static agent to form a dry mix blend. Specifically, in part I, 20.75 mg of mannitol granular 200SD is sifted through 36 mesh of the vibratory sifter to obtain agglomerate free mass of sifted mannitol granular 200SD. Thereafter, in part II, 10 mg of lisdexamfetamine dimesylate, 15 mg of partially pregelatinized maize starch, and 1.50 mg of colloidal anhydrous silica are co-sifted through 36 mesh of the vibratory sifter to obtain agglomerate free mass of sifted lisdexamfetamine dimesylate, partially pregelatinized maize starch and colloidal anhydrous silica.

Thereafter, as a first level of mixing, the agglomerate free mass of sifted lisdexamfetamine dimesylate, partially pregelatinized maize starch and colloidal anhydrous silica obtained at the previous step is blended in a conta blender at 8 RPM for 30 minutes to form blended mixture of lisdexamfetamine dimesylate, partially pregelatinized maize starch and colloidal anhydrous silica.

Thereafter, as a second level of mixing, the blended mixture is further blended in the conta blender at 8 RPM for 15 minutes to obtain blended material. Thereafter, the blended material is unloaded in HDPE container lined with polyethylene bag and added with agglomerate free mass of sifted mannitol granular 200SD to form a dry mix blend.

In the next step, the filler, the cushioning agent, the disintegrating agent, and the sweetener are blended to form an intermediate granular blend.

Specifically, 31 mg of mannitol granular 200SD, 10 mg of microcrystalline cellulose, 8 mg of crospovidone, and 2.50 mg of sucralose are co-sifted through 36 mesh of the vibratory sifter to obtain agglomerate free mass of sifted mannitol granular 200SD, microcrystalline cellulose, crospovidone, and sucralose.

Thereafter, agglomerate free mass of sifted mannitol granular 200SD, microcrystalline cellulose, crospovidone, and sucralose obtained at the previous step is blended in the conta blender at 8 RPM for 15 minutes to obtain intermediate granular blend. The intermediate granular blend obtained at this step is further divided into two parts as first half of the intermediate granular blend and second half of the intermediate granular blend. The first half of the intermediate granular blend is unloaded in the HDPE container lined with polybag, the second half of the intermediate granular blend is left in the bin of the conta blender.

The next step in the method of preparation involves adding and blending the dry mix blend and the intermediate granular blend to form an intermediate drug blend. In this step, the dry mix blend is added to the second half of the intermediate granular blend followed by addition of the first half of the intermediate granular blend. After addition of the second half of the intermediate granular blend; dry mix blend; and the first half of the intermediate granular blend sequentially to the bin of the conta blender, the mixture is blended at 8 RPM for 30 minutes to obtain intermediate drug blend.

The next step in the method involves lubricating the intermediate drug blend with magnesium stearate to form ready to compress drug granules. Herein, 1.25 mg of magnesium stearate is sifted through 40 mesh of the vibratory sifter to obtain agglomerate free mass of the sifted magnesium stearate. Further, the sifted magnesium stearate is added to the intermediate drug blend obtained at previous step and blended in the conta blender at 8 RPM for 10 minutes to obtain ready to compress drug granules.

Thereafter, ready to compress drug granules are compressed by employing direct compression technique involving controlled humidity level of NMT 40% to reduce exposure of moisture while forming 10 mg of lisdexamfetamine dimesylate dispersible tablet.

In TABLE 4A, the product description of the compressed Lisdexamfetamine Dimesylate (10 mg) dispersible tablet is depicted.

**TABLE 4A**

| **PRODUCT DESCRIPTION** | |
|---|---|
| Tablet Weight | 100 mg ± 7.5% |
| Hardness | 30 N |
| Thickness | 2.2 mm ±0.3 mm |
| Diameter | 7 mm ±0.3 mm |
| Disintegration Time (DT) | NMT 3 min at 15°C to 25°C (without disc) |
| Friability | NMT 1.5% |

In one embodiment, for Lisdexamfetamine Dimesylate (10 mg) dispersible tablet, the disintegration time is 58 seconds.

In TABLE 4B, the physical characteristics of the Lisdexamfetamine Dimesylate (10 mg) dispersible tablet are depicted.

**TABLE 4B**

| **Product Name** | Lisdexamfetamine Dimesylate dispersible tablet (10 mg) |
|---|---|
| Appearance (Color) | White to off-white |
| Shape | Flat beveled edge round, plain on both surfaces |
| Size (Thickness) | 2.2 mm ±0.3 mm |

### EXAMPLE 2

In Example 2, the method for preparation of lisdexamfetamine dimesylate dispersible tablet is described. Particularly, the method for preparation is described for preparing lisdexamfetamine dimesylate dispersible tablet having strength of 20 mg per tablet.

In TABLE 5, the amount of API and excipients (in mg/tablet) for Lisdexamfetamine Dimesylate (20 mg) dispersible tablets are depicted.

**TABLE 5**

| **DRY MIXING** | |
|---|---|
| Ingredients | Relative Qty. (in mg/tablet) |
| Lisdexamfetamine dimesylate | 20.0 mg |
| Mannitol Granular 200SD (Pearlitol^{®}200SD) | 41.50 mg |
| Partially pregelatinized Maize Starch (Starch1500^{®}) | 30 mg |
| Colloidal Anhydrous Silica (Aerosil^{®}200) | 3 mg |

| **INTERMEDIATE BLENDING** | |
|---|---|
| Mannitol Granular 200SD (Pearlitol^{®}200SD) | 62 mg |
| Microcrystalline Cellulose (VIVAPUR^{®} 112) | 20 mg |
| Crospovidone (Kollidone^{®}CL) | 16 mg |
| Sucralose | 5 mg |

| **LUBRICATION** | |
|---|---|
| Magnesium Stearate | 2.50 mg |
| Total | 200.0 mg |

The method is initiated by dry mixing lisdexamfetamine dimesylate, a diluent, a binder and an anti-static agent to form a dry mix blend. Specifically, in part I, 41.50 mg of mannitol granular 200SD is sifted through 36 mesh of the vibratory sifter to obtain agglomerate free mass of sifted mannitol granular 200SD. Thereafter, in part II, 20 mg of lisdexamfetamine dimesylate, 30 mg of partially pregelatinized maize starch, and 3 mg colloidal anhydrous silica are co-sifted through 36 mesh of the vibratory sifter to obtain agglomerate free mass of sifted lisdexamfetamine dimesylate, partially pregelatinized maize starch and colloidal anhydrous silica.

Thereafter, as a first level of mixing, the agglomerate free mass of sifted lisdexamfetamine dimesylate, partially pregelatinized maize starch and colloidal anhydrous silica obtained at the previous step is blended in a conta blender at 8 RPM for 30 minutes to form blended mixture of lisdexamfetamine dimesylate, partially pregelatinized maize starch and colloidal anhydrous silica.

Thereafter, as a second level of mixing, the blended mixture is further blended in the conta blender at 8 RPM for 15 minutes to obtain blended material. Thereafter, the blended material is unloaded in HDPE container lined with polyethylene bag and added with agglomerate free mass of sifted mannitol granular 200SD to form dry mix blend.

As a next step in the method of preparation, the filler, cushioning agent, disintegrating agent, and sweetener are blended to form intermediate granular blend.

Specifically, 62 mg of mannitol granular 200SD, 20 mg of microcrystalline cellulose, 16 mg of crospovidone, and 5 mg of sucralose are co-sifted through 36 mesh of the vibratory sifter to obtain agglomerate free mass of the sifted mannitol granular 200SD, microcrystalline cellulose, crospovidone, and sucralose.

Thereafter, agglomerate free mass of sifted mannitol granular 200SD, microcrystalline cellulose, crospovidone, and sucralose obtained at the previous step is blended in the conta blender at 8 RPM for 15 minutes to obtain intermediate granular blend. The intermediate granular blend obtained at this step is further divided into two parts as first half of the intermediate granular blend and second half of the intermediate granular blend. The first half of the intermediate granular blend is unloaded in the HDPE container lined with polybag, the second half of the intermediate granular blend is left in the bin of the conta blender.

The next step in the method of preparation involves adding and blending the dry mix blend and the intermediate granular blend to form an intermediate drug blend. In this step, the dry mix blend is added to the second half of the intermediate granular blend followed by addition of the first half of the intermediate granular blend. After addition of the second half of the intermediate granular blend; dry mix blend; and the first half of the intermediate granular blend sequentially to the bin of the conta blender, the mixture is blended at 8 RPM for 30 minutes to obtain intermediate drug blend.

The next step in the method of preparation involves lubricating the intermediate drug blend with magnesium stearate to form ready to compress drug granules. Herein, 2.50 mg of magnesium stearate is sifted through 40 mesh of the vibratory sifter to obtain agglomerate free mass of the sifted magnesium stearate. Further, the sifted magnesium stearate is added to the intermediate drug blend obtained at previous step and blended in the conta blender at 8 RPM for 10 minutes to obtain ready to compress drug granules.

Thereafter, ready to compress drug granules are compressed by employing direct compression technique involving controlled humidity level of NMT 40% to reduce exposure of moisture while forming 20 mg of lisdexamfetamine dimesylate dispersible tablet.

In TABLE 6A, the product description of the compressed Lisdexamfetamine Dimesylate (20 mg) dispersible tablet is depicted.

**TABLE 6A**

| **PRODUCT DESCRIPTION** | |
|---|---|
| Tablet Weight | 200 mg ± 7.5% |
| Hardness | 35 N |
| Thickness | 3.6 mm ±0.3 mm |
| Diameter | 8 mm ±0.3 mm |
| Disintegration Time (DT) | NMT 3 min at 15°C to 25°C (without disc) |
| Friability | NMT 1.5 % |

In one embodiment, for compressed Lisdexamfetamine Dimesylate (20 mg) dispersible tablet, the disintegration time is 35 seconds.

In TABLE 6B, the physical characteristics of the compressed Lisdexamfetamine Dimesylate (20 mg) dispersible tablet are depicted.

**TABLE 6B**

| **Product Name** | Lisdexamfetamine Dimesylate dispersible tablet (20 mg) |
|---|---|
| Appearance (Color) | White to off-white |
| Shape | Flat beveled edge round, plain on both surfaces |
| Size (Thickness) | 3.6 mm ±0.3 mm |

### EXAMPLE 3

In Example 3, the method for preparation of lisdexamfetamine dimesylate dispersible tablet is described. Particularly, the method for preparation is described for preparing lisdexamfetamine dimesylate dispersible tablet having strength of 30 mg per tablet.

In TABLE 7, the amount of API and excipients (in mg/tablet) for Lisdexamfetamine Dimesylate (30 mg) dispersible tablets are depicted.

**TABLE 7**

| **DRY MIXING** | |
|---|---|
| Ingredients | Relative Qty. (in mg/tablet) |
| Lisdexamfetamine dimesylate | 30.0 mg |
| Mannitol Granular 200SD (Pearlitol^{®}200SD) | 62.25 mg |
| Partially pregelatinized Maize Starch (Starch1500^{®}) | 45.0 mg |
| Colloidal anhydrous Silica (Aerosil^{®} 200) | 4.5 mg |

| **INTERMEDIATE BLENDING** | |
|---|---|
| Mannitol Granular 200SD (Pearlitol^{®} 200SD) | 93.0 mg |
| Microcrystalline Cellulose (VIVAPUR^{®} 112) | 30.0 mg |
| Crospovidone (Kollidone^{®}CL) | 24.0 mg |
| Sucralose | 7.5 mg |

| **LUBRICATION** | |
|---|---|
| Magnesium Stearate | 3.75 mg |
| Total | 300.0 Mg |

The method is initiated by dry mixing lisdexamfetamine dimesylate, a diluent, a binder and an anti-static agent to form a dry mix blend. Specifically, in part I, 62.25 mg of mannitol granular 200SD is sifted through 36 mesh of the vibratory sifter to obtain agglomerate free mass of sifted mannitol granular 200SD. Thereafter, in part II, 30 mg of lisdexamfetamine dimesylate, 45 mg of partially pregelatinized maize starch, and 4.50 mg of colloidal anhydrous silica are co-sifted through 36 mesh of the vibratory sifter to obtain agglomerate free mass of sifted lisdexamfetamine dimesylate, partially pregelatinized maize starch and colloidal anhydrous silica.

Thereafter, as a first level of mixing, the agglomerate free mass of sifted lisdexamfetamine dimesylate, partially pregelatinized maize starch and colloidal anhydrous silica obtained at the previous step is blended in a conta blender at 8 RPM for 30 minutes to form blended mixture of lisdexamfetamine dimesylate, partially pregelatinized maize starch and colloidal anhydrous silica.

Thereafter, as a second level of mixing, the blended mixture is further blended in the conta blender at 8 RPM for 15 minutes to obtain blended material. Thereafter, the blended material is unloaded in HDPE container lined with polyethylene bag and added with agglomerate free mass of mannitol granular 200SD to form dry mix blend.

In the next step, the filler, the cushioning agent, the disintegrating agent, and the sweetener are blended to form intermediate granular blend.

Specifically, 93 mg of mannitol granular 200SD, 30 mg of microcrystalline cellulose, 24 mg of crospovidone, and 7.50 mg of sucralose are co-sifted through 36 mesh of the vibratory sifter to obtain agglomerate free mass of the sifted mannitol granular 200SD, microcrystalline cellulose, crospovidone, and sucralose.

Thereafter, agglomerate free mass of the sifted mannitol granular 200SD, microcrystalline cellulose, crospovidone, and sucralose obtained at the previous step is blended in the conta blender at 8 RPM for 15 minutes to obtain intermediate granular blend. The intermediate granular blend obtained at this step is further divided into two parts as first half of the intermediate granular blend and second half of the intermediate granular blend. The first half of the intermediate granular blend is unloaded in the HDPE container lined with polybag, the second half of the intermediate granular blend is left in the bin of the conta blender.

The next step in the method involves adding and blending the dry mix blend and the intermediate granular blend to form an intermediate drug blend. In this step, the dry mix blend is added to the second half of the intermediate granular blend followed by addition of the first half of the intermediate granular blend. After addition of the second half of the intermediate granular blend; dry mix blend; and the first half of the intermediate granular blend sequentially to the bin of the conta blender, the mixture is blended at 8 RPM for 30 minutes to obtain intermediate drug blend.

The next step in the method of preparation involves lubricating the intermediate drug blend with magnesium stearate to form ready to compress drug granules. Herein, 3.75 mg of magnesium stearate is sifted through 40 mesh of the vibratory sifter to obtain agglomerate free mass of the sifted magnesium stearate. Further, the sifted magnesium stearate is added to the intermediate drug blend obtained at previous step and blended in the conta blender at 8 RPM for 10 minutes to obtain ready to compress drug granules.

Thereafter, ready to compress drug granules are compressed by employing direct compression technique involving controlled humidity level of NMT 40% to reduce exposure of moisture while forming 30 mg of lisdexamfetamine dimesylate dispersible tablet.

In TABLE 8A, the amount of API and excipients (in mg/tablet) for Lisdexamfetamine Dimesylate (30 mg) dispersible tablets are depicted.

**TABLE 8A**

| **PRODUCT DESCRIPTION** | |
|---|---|
| Tablet Weight | 300 mg ± 5% |
| Hardness | 40 N |
| Thickness | 3.50 mm ±0.3 mm |
| Diameter | 10 mm ±0.3 mm |
| Disintegration Time (DT) | NMT 3 min at 15°C to 25°C (without disc) |
| Friability | NMT 1.5% |

In one embodiment, for compressed Lisdexamfetamine Dimesylate (30 mg) dispersible tablet, the disintegration time is 30 seconds.

In TABLE 8B, the physical characteristics of the compressed Lisdexamfetamine Dimesylate (30 mg) dispersible tablet are depicted.

**TABLE 8B**

| **Product Name** | Lisdexamfetamine Dimesylate dispersible tablet (30 mg) |
|---|---|
| Appearance (Color) | White to off-white |
| Shape | Flat beveled edge round, plain on both surfaces |
| Size (Thickness) | 3.50 mm ±0.3 mm |

### EXAMPLE 4

In Example 4, the method for preparation of lisdexamfetamine dimesylate dispersible tablet is described. Particularly, the method for preparation is described for preparing lisdexamfetamine dimesylate dispersible tablet having strength of 40 mg per tablet.

In TABLE 9, the amount of API and excipients (in mg/tablet) for Lisdexamfetamine Dimesylate (40 mg) dispersible tablets are depicted.

**TABLE 9**

| **DRY MIXING** | |
|---|---|
| Ingredients | Relative Qty. (in mg/tablet) |
| Lisdexamfetamine dimesylate | 40.0 mg |
| Mannitol Granular 200SD (Pearlitol^{®}200SD) | 83.0 mg |
| Partially pregelatinized Maize Starch (Starch1500^{®}) | 60.0 mg |
| Colloidal anhydrous Silica (Aerosil^{®} 200) | 6.0 mg |

| **INTERMEDIATE BLENDING** | |
|---|---|
| Mannitol Granular 200SD (Pearlitol^{®} 200SD) | 124.0 mg |
| Microcrystalline Cellulose (VIVAPUR^{®} 112) | 40.0 mg |
| Crospovidone (Kollidone^{®}CL) | 32.0 mg |
| Sucralose | 10.0 mg |

| **LUBRICATION** | |
|---|---|
| Magnesium Stearate | 5.0 mg |
| Total | 400.0 mg |

The method is initiated by dry mixing lisdexamfetamine dimesylate, a diluent, a binder and an anti-static agent to form a dry mix blend. Specifically, in part I, 83 mg of mannitol granular 200SD is sifted through 36 mesh of the vibratory sifter to obtain agglomerate free mass of sifted mannitol granular 200SD. Thereafter, in part II, 40 mg of lisdexamfetamine dimesylate, 60 mg of partially pregelatinized maize starch, and 6 mg colloidal anhydrous silica are co-sifted through 36 mesh of the vibratory sifter to obtain agglomerate free mass of sifted lisdexamfetamine dimesylate, partially pregelatinized maize starch and colloidal anhydrous silica.

Thereafter, as a first level of mixing, the agglomerate free mass of sifted lisdexamfetamine dimesylate, partially pregelatinized maize starch and colloidal anhydrous silica obtained at the previous step is blended in a conta blender at 8 RPM for 30 minutes to form blended mixture of lisdexamfetamine dimesylate, partially pregelatinized maize starch and colloidal anhydrous silica.

Thereafter, as a second level of mixing, the blended mixture is further blended in the conta blender at 8 RPM for 15 minutes to obtain blended material. Thereafter, the blended material is unloaded in HDPE container lined with polyethylene bag and added with agglomerate free mass of mannitol granular 200SD to form dry mix blend.

In the next step, the filler, the cushioning agent, the disintegrating agent, and the sweetener are blended to form intermediate granular blend.

Specifically, 124 mg of mannitol granular 200SD, 40 mg of microcrystalline cellulose, 32 mg of crospovidone, and 10 mg of sucralose are co-sifted through 36 mesh of the vibratory sifter to obtain agglomerate free mass of the sifted mannitol granular 200SD, microcrystalline cellulose, crospovidone, and sucralose.

Thereafter, agglomerate free mass of the sifted mannitol granular 200SD, microcrystalline cellulose, crospovidone, and sucralose obtained at the previous step is blended in the conta blender at 8 RPM for 15 minutes to obtain intermediate granular blend. The intermediate granular blend obtained at this step is further divided into two parts as first half of the intermediate granular blend and second half of the intermediate granular blend. The first half of the intermediate granular blend is unloaded in the HDPE container lined with polybag, the second half of the intermediate granular blend is left in the bin of the conta blender.

The next step in the method of preparation involves adding and blending the dry mix blend and the intermediate granular blend to form an intermediate drug blend. In this step, the dry mix blend is added to the second half of the intermediate granular blend followed by addition of the first half of the intermediate granular blend. After addition of the second half of the intermediate granular blend; dry mix blend; and the first half of the intermediate granular blend sequentially to the bin of the conta blender, the mixture is blended at 8 RPM for 30 minutes to obtain intermediate drug blend.

The next step in the method of preparation involves lubricating the intermediate drug blend with magnesium stearate to form ready to compress drug granules. Herein, 5 mg of magnesium stearate is sifted through 40 mesh of the vibratory sifter to obtain agglomerate free mass of the sifted magnesium stearate. Further, the sifted magnesium stearate is added to the intermediate drug blend obtained at previous step and blended in the conta blender at 8 RPM for 10 minutes to obtain ready to compress drug granules.

Thereafter, ready to compress drug granules are compressed by employing direct compression technique involving controlled humidity level of NMT 40% to reduce exposure of moisture while forming 40 mg of lisdexamfetamine dimesylate dispersible tablet.

In TABLE 10A, the product description of the compressed Lisdexamfetamine Dimesylate (40 mg) dispersible tablet is depicted.

**TABLE 10A**

| **PRODUCT DESCRIPTION** | |
|---|---|
| Tablet Weight | 400 mg ± 5% |
| Hardness | 50 N |
| Thickness | 3.80 mm ±0.3 mm |
| Diameter | 11 mm ±0.3 mm |
| Disintegration Time (DT) | NMT 3 min at 15°C to 25°C (without disc) |
| Friability | NMT 1.5% |

In one embodiment, for compressed Lisdexamfetamine Dimesylate (40 mg) dispersible tablet, the disintegration time is 45 seconds.

In TABLE 10B, the physical characteristics of the compressed Lisdexamfetamine Dimesylate (40 mg) dispersible tablet are depicted.

**TABLE 10B**

| **Product Name** | lisdexamfetamine dimesylate dispersible tablet (40 mg) |
|---|---|
| Appearance (Color) | White to off-white |
| Shape | Flat beveled edge round, plain on both surfaces |
| Size (Thickness) | 3.80 mm ±0.3 mm |

### EXAMPLE 5

In Example 5, the method for preparation of lisdexamfetamine dimesylate dispersible tablet is described. Particularly, the method for preparation is described for preparing lisdexamfetamine dimesylate dispersible tablet having strength of 50 mg per tablet

In TABLE 11, the amount of API and excipients (in mg/tablet) for Lisdexamfetamine Dimesylate (50 mg) dispersible tablets are depicted.

**TABLE 11**

| **DRY MIXING** | |
|---|---|
| Ingredients | Relative Qty. (in mg/tablet) |
| Lisdexamfetamine dimesylate | 50.0 mg |
| Mannitol Granular 200SD (Pearlitol^{®}200SD) | 103.75 mg |
| Partially pregelatinized Maize Starch (Starch1500^{®}) | 75.0 mg |
| Colloidal anhydrous Silica (Aerosil^{®} 200) | 7.50 mg |

| **INTERMEDIATE BLENDING** | |
|---|---|
| Mannitol Granular 200SD (Pearlitol^{®} 200SD) | 155.0 mg |
| Microcrystalline Cellulose (VIVAPUR^{®} 112) | 50.0 mg |
| Crospovidone (Kollidone^{®}CL) | 40.0 mg |
| Sucralose | 12.5 mg |

| **LUBRICATION** | |
|---|---|
| Magnesium Stearate | 6.25 mg |
| Total | 500.0 mg |

The method is initiated by dry mixing lisdexamfetamine dimesylate, a diluent, a binder and an anti-static agent to form a dry mix blend. Specifically, in part I, 103.75 mg of mannitol granular 200SD is sifted through 36 mesh of the vibratory sifter to obtain agglomerate free mass of sifted mannitol granular 200SD. Thereafter, in part II, 50 mg of lisdexamfetamine dimesylate, 75 mg of partially pregelatinized maize starch, and 7.5 mg of colloidal anhydrous silica are co-sifted through 36 mesh of the vibratory sifter to obtain agglomerate free mass of sifted lisdexamfetamine dimesylate, partially pregelatinized maize starch and colloidal anhydrous silica.

Thereafter, as a first level of mixing, the agglomerate free mass of sifted lisdexamfetamine dimesylate, partially pregelatinized maize starch and colloidal anhydrous silica obtained at the previous step is blended in a conta blender at 8 RPM for 30 minutes to form blended mixture of lisdexamfetamine dimesylate, partially pregelatinized maize starch and colloidal anhydrous silica.

Thereafter, as a second level of mixing, the blended mixture is further blended in the conta blender at 8 RPM for 15 minutes to obtain blended material. Thereafter, the blended material is unloaded in HDPE container lined with polyethylene bag and added with agglomerate free mass of sifted mannitol granular 200SD to form dry mix blend.

In the next step, the filler, the cushioning agent, the disintegrating agent, and the sweetener are blended to form intermediate granular blend.

Specifically, 155 mg of mannitol granular 200SD, 50 mg of microcrystalline cellulose, 40 mg of crospovidone, and 12.5 mg sucralose are co-sifted through 36 mesh of the vibratory sifter to obtain agglomerate free mass of the sifted mannitol granular 200SD, microcrystalline cellulose, crospovidone, and sucralose.

Thereafter, agglomerate free mass of the sifted mannitol granular 200SD, microcrystalline cellulose, crospovidone, and sucralose obtained at the previous step is blended in the conta blender at 8 RPM for 15 minutes to obtain intermediate granular blend. The intermediate granular blend obtained at this step is further divided into two parts as first half of the intermediate granular blend and second half of the intermediate granular blend. The first half of the intermediate granular blend is unloaded in the HDPE container lined with polybag, the second half of the intermediate granular blend is left in the bin of the conta blender.

The next step in the method of preparation involves adding and blending the dry mix blend and the intermediate granular blend to form an intermediate drug blend. In this step, the dry mix blend is added to the second half of the intermediate granular blend followed by addition of the first half of the intermediate granular blend. After addition of the second half of the intermediate granular blend; dry mix blend; and the first half of the intermediate granular blend sequentially to the bin of the conta blender, the mixture is blended at 8 RPM for 30 minutes to obtain intermediate drug blend.

The next step in the method of preparation involves lubricating the intermediate drug blend with magnesium stearate to form ready to compress drug granules. Herein, 6.25 mg of magnesium stearate is sifted through 40 mesh of the vibratory sifter to obtain agglomerate free mass of the sifted magnesium stearate. Further, the sifted magnesium stearate is added to the intermediate drug blend obtained at previous step and blended in the conta blender at 8 RPM for 10 minutes to obtain ready to compress drug granules.

Thereafter, ready to compress drug granules are compressed by employing direct compression technique involving controlled humidity level of NMT 40% to reduce exposure of moisture while forming 50 mg of lisdexamfetamine dimesylate dispersible tablet.

In TABLE 12A, the product description of the compressed Lisdexamfetamine Dimesylate (50 mg) dispersible tablet is depicted.

**TABLE 12A**

| **PRODUCT DESCRIPTION** | |
|---|---|
| Tablet Weight | 500 mg ± 5% |
| Hardness | 50 N |
| Thickness | 4.0 mm ±0.3 mm |
| Diameter | 12 mm ±0.3 mm |
| Disintegration Time (DT) | NMT 3 min at 15°C to 25°C (without disc) |
| Friability | NMT 1.5% |

In one embodiment, for compressed Lisdexamfetamine Dimesylate (50 mg) dispersible tablet, the disintegration time is 48 seconds.

In TABLE 12B, the physical characteristics of the compressed Lisdexamfetamine Dimesylate (50 mg) dispersible tablet are depicted.

**TABLE 12B**

| **Product Name** | Lisdexamfetamine Dimesylate dispersible tablet (50 mg) |
|---|---|
| Appearance (Color) | White to off-white |
| Shape | Flat beveled edge round, plain on both surfaces |
| Size (Thickness) | 4.0 mm ±0.3 mm |

### EXAMPLE 6

In Example 6, the method for preparation of lisdexamfetamine dimesylate dispersible tablet is described. Particularly, the method for preparation is described for preparing lisdexamfetamine dimesylate dispersible tablet having strength of 60 mg per tablet.

In TABLE 13, the amount of API and excipients (in mg/tablet) for Lisdexamfetamine Dimesylate (60 mg) dispersible tablets are depicted.

**TABLE 13**

| **DRY MIXING** | |
|---|---|
| Ingredients | Relative Qty. (in mg/tablet) |
| Lisdexamfetamine dimesylate | 60.0 mg |
| Mannitol Granular 200SD (Pearlitol^{®}200SD) | 124.50 mg |
| Partially pregelatinized Maize Starch (Starch1500^{®}) | 90.0 mg |
| Colloidal anhydrous Silica (Aerosil^{®} 200) | 9.0 mg |

| **INTERMEDIATE BLENDING** | |
|---|---|
| Mannitol Granular 200SD (Pearlitol^{®} 200SD) | 186 mg |
| Microcrystalline Cellulose (VIVAPUR^{®} 112) | 60.0 mg |
| Crospovidone (Kollidone^{®}CL) | 48.0 mg |
| Sucralose | 15.0 mg |

| **LUBRICATION** | |
|---|---|
| Magnesium Stearate | 7.5 mg |
| Total | 600.0 mg |

The method is initiated by dry mixing lisdexamfetamine dimesylate, a diluent, a binder and an anti-static agent to form a dry mix blend. Specifically, in part I, 124.50 mg of mannitol granular 200SD is sifted through 36 mesh of the vibratory sifter to obtain agglomerate free mass of sifted mannitol granular 200SD. Thereafter, in part II, 60 mg of lisdexamfetamine dimesylate, 90 mg of partially pregelatinized maize starch, and 9 mg of colloidal anhydrous silica are co-sifted through 36 mesh of the vibratory sifter to obtain agglomerate free mass of sifted lisdexamfetamine dimesylate, partially pregelatinized maize starch and colloidal anhydrous silica.

Thereafter, as a first level of mixing, the agglomerate free mass of sifted lisdexamfetamine dimesylate, partially pregelatinized maize starch and colloidal anhydrous silica obtained at the previous step is blended in a conta blender at 8 RPM for 30 minutes to form blended mixture of lisdexamfetamine dimesylate, partially pregelatinized maize starch and colloidal anhydrous silica.

Thereafter, as a second level of mixing, the blended mixture is further blended in the conta blender at 8 RPM for 15 minutes to obtain blended material. Thereafter, the blended material is unloaded in HDPE container lined with polyethylene bag and added with agglomerate free mass of sifted mannitol granular 200SD to form dry mix blend.

In the next step the filler, the cushioning agent, the disintegrating agent, and the sweetener are blended to form intermediate granular blend.

Specifically, 186 mg of mannitol granular 200SD, 60 mg of microcrystalline cellulose, 48 mg of crospovidone, and 15 mg of sucralose are co-sifted through 36 mesh of the vibratory sifter to obtain agglomerate free mass of the sifted mannitol granular 200SD, microcrystalline cellulose, crospovidone, and sucralose.

Thereafter, agglomerate free mass of the sifted mannitol granular 200SD, microcrystalline cellulose, crospovidone, and sucralose obtained at the previous step is blended in the conta blender at 8 RPM for 15 minutes to obtain intermediate granular blend. The intermediate granular blend obtained at this step is further divided into two parts as first half of the intermediate granular blend and second half of the intermediate granular blend. The first half of the intermediate granular blend is unloaded in the HDPE container lined with polybag, the second half of the intermediate granular blend is left in the bin of the conta blender.

The next step in the method of preparation involves adding and blending the dry mix blend and the intermediate granular blend to form an intermediate drug blend. In this step, the dry mix blend is added to the second half of the intermediate granular blend followed by addition of the first half of the intermediate granular blend. After addition of the second half of the intermediate granular blend; dry mix blend; and the first half of the intermediate granular blend sequentially to the bin of the conta blender, the mixture is blended at 8 RPM for 30 minutes to obtain intermediate drug blend.

The next step in the method of preparation involves lubricating the intermediate drug blend with magnesium stearate to form ready to compress drug granules. Herein, 7.50 mg of magnesium stearate is sifted through 40 mesh of the vibratory sifter to obtain agglomerate free mass of the sifted magnesium stearate. Further, the sifted magnesium stearate is added to the intermediate drug blend obtained at previous step and blended in the conta blender at 8 RPM for 10 minutes to obtain ready to compress drug granules.

Thereafter, ready to compress drug granules are compressed by employing direct compression technique involving controlled humidity level of NMT 40% to reduce exposure of moisture while forming 60 mg of lisdexamfetamine dimesylate dispersible tablet.

In TABLE 14A, the product description of the compressed Lisdexamfetamine Dimesylate (60 mg) dispersible tablet is depicted.

**TABLE 14A**

| **PRODUCT DESCRIPTION** | |
|---|---|
| Tablet Weight | 600 mg ± 5% |
| Hardness | 60 N |
| Thickness | 4.1 mm ±0.3 mm |
| Diameter | 13 mm ±0.3 mm |
| Disintegration Time (DT) | NMT 3 min at 15°C to 25°C (without disc) |
| Friability | NMT 1.5% |

In one embodiment, for compressed Lisdexamfetamine Dimesylate (60 mg) dispersible tablet, the disintegration time is 48 seconds.

In TABLE 14B, the physical characteristics of the compressed Lisdexamfetamine Dimesylate (60 mg) dispersible tablet are depicted.

**TABLE 14B**

| **Product Name** | Lisdexamfetamine Dimesylate dispersible tablet (60 mg) |
|---|---|
| Appearance (Color) | White to off-white |
| Shape | Flat beveled edge round, plain on both surfaces |
| Size (Thickness) | 4.1 mm ±0.3 mm |

### EXAMPLE 7

In Example 7, the method for preparation of lisdexamfetamine dimesylate dispersible tablet is described. Particularly, the method for preparation is described for preparing lisdexamfetamine dimesylate dispersible tablet having strength of 70 mg per tablet.

In TABLE 15, the amount of API and excipients (in mg/tablet) for Lisdexamfetamine Dimesylate (70 mg) dispersible tablets are depicted.

**TABLE 15**

| **DRY MIXING** | |
|---|---|
| Ingredients | Relative Qty. (in mg/tablet) |
| Lisdexamfetamine dimesylate | 70.0 mg |
| Mannitol Granular 200SD (Pearlitol^{®}200SD) | 145.25 mg |
| Partially pregelatinized Maize Starch (Starch1500^{®}) | 105.0 mg |
| Colloidal anhydrous Silica (Aerosil^{®} 200) | 10.50 mg |

| **INTERMEDIATE BLENDING** | |
|---|---|
| Mannitol Granular 200SD (Pearlitol^{®}200SD) | 217.0 mg |
| Microcrystalline Cellulose (VIVAPUR^{®} 112) | 70.0 mg |
| Crospovidone (Kollidone^{®}CL) | 56.0 mg |
| Sucralose | 17.5 mg |

| **LUBRICATION** | |
|---|---|
| Magnesium Stearate | 8.75 mg |
| Total | 700.0 mg |

The method is initiated by dry mixing lisdexamfetamine dimesylate, a diluent, a binder and an anti-static agent to form a dry mix blend. Specifically, in part I, 145.25 mg of mannitol granular 200SD is sifted through 36 mesh of the vibratory sifter to obtain agglomerate free mass of sifted mannitol granular 200SD. Thereafter, in part II, 70 mg of lisdexamfetamine dimesylate, 105 mg of partially pregelatinized maize starch, and 10.50 mg of colloidal anhydrous silica are co-sifted through 36 mesh of the vibratory sifter to obtain agglomerate free mass of sifted lisdexamfetamine dimesylate, partially pregelatinized maize starch and colloidal anhydrous silica.

Thereafter, as a first level of mixing, the agglomerate free mass of sifted lisdexamfetamine dimesylate, partially pregelatinized maize starch and colloidal anhydrous silica obtained at the previous step is blended in a conta blender at 8 RPM for 30 minutes to form blended mixture of lisdexamfetamine dimesylate, partially pregelatinized maize starch and colloidal anhydrous silica.

Thereafter, as a second level of mixing, the blended mixture is further blended in the conta blender at 8 RPM for 15 minutes to obtain blended material. Thereafter, the blended material is unloaded in HDPE container lined with polyethylene bag and added with agglomerate free mass of mannitol granular 200SD to form dry mix blend.

As a next step in the method of preparation, the filler, cushioning agent, disintegrating agent, and sweetener are blended to form intermediate granular blend.

Specifically, 217 mg of mannitol granular 200SD, 70 mg of microcrystalline cellulose, 56 mg of crospovidone, and 17.50 mg sucralose are co-sifted through 36 mesh of the vibratory sifter to obtain agglomerate free mass of the sifted mannitol granular 200SD, microcrystalline cellulose, crospovidone, and sucralose.

Thereafter, agglomerate free mass of the sifted mannitol granular 200SD, microcrystalline cellulose, crospovidone, and sucralose obtained at the previous step is blended in the conta blender at 8 RPM for 15 minutes to obtain intermediate granular blend. The intermediate granular blend obtained at this step is further divided into two parts as first half and second half of the intermediate granular blend. The first half of the intermediate granular blend is unloaded in the HDPE container lined with polybag, the second half of the intermediate granular blend is left in the bin of the conta blender.

The next step in the method of preparation involves adding and blending the dry mix blend and the intermediate granular blend to form an intermediate drug blend. In this step, the dry mix blend is added to the second half of the intermediate granular blend followed by addition of the first half of the intermediate granular blend. After addition of the second half of the intermediate granular blend; dry mix blend; and the first half of the intermediate granular blend sequentially to the bin of the conta blender, the mixture is blended at 8 RPM for 30 minutes to obtain intermediate drug blend.

The next step in the method of preparation involves lubricating the intermediate drug blend with magnesium stearate to form ready to compress drug granules. Herein, 8.75 mg of magnesium stearate is sifted through 40 mesh of the vibratory sifter to obtain agglomerate free mass of the sifted magnesium stearate. Further, the sifted magnesium stearate is added to the intermediate drug blend obtained at previous step and blended in the conta blender at 8 RPM for 10 minutes to obtain ready to compress drug granules.

Thereafter, ready to compress drug granules are compressed by employing direct compression technique involving controlled humidity level of NMT 40% to reduce exposure of moisture while forming 70 mg of lisdexamfetamine dimesylate dispersible tablet.

In TABLE 16A, the product description of the compressed Lisdexamfetamine Dimesylate (70 mg) dispersible tablet is depicted.

**TABLE 16A**

| **PRODUCT DESCRIPTION** | |
|---|---|
| Tablet Weight | 700 mg ± 5% |
| Hardness | 70 N |
| Thickness | 4.0 mm ±0.3 mm |
| Diameter | 14 mm ±0.3 mm |
| Disintegration Time (DT) | NMT 3 min at 15°C to 25°C (without disc) |
| Friability | NMT 1.5% |

In one embodiment, for compressed Lisdexamfetamine Dimesylate (70 mg) dispersible tablet, the disintegration time is 46 seconds.

In TABLE 16B, the physical characteristics of the compressed Lisdexamfetamine Dimesylate (70 mg) dispersible tablet are depicted.

**TABLE 16B**

| **Product Name** | lisdexamfetamine dimesylate dispersible tablet (70 mg) |
|---|---|
| Appearance (Color) | White to off-white |
| Shape | Flat beveled edge round, plain on both surfaces |
| Size (Thickness) | 4.0 mm ±0.3 mm |

### Tests:

The included data for the test results shows readings with regard to moisture-controlled tablet composition, i.e., tablet in which the deliquescence of API is taken care of, in other words it shows data on how the exact combination of drug along with all different excipients lead to a formulation/composition with no or insignificant deliquescence.

In order to demonstrate the composition comprising lisdexamfetamine dimesylate does not exhibit deliquescent properties, a series of tests have been performed aimed at assessing the stability, durability, and resistance to various forms of degradation of the tablet composition comprising lisdexamfetamine dimesylate.

The tests are accelerated aging test to assess its resistance to degradation over time; chemical resistance test / compatibility test to assess suitability of excipients materials with the lisdexamfetamine dimesylate tablets; moisture absorption test to show no significant increase in absorption of moisture level by the tablet comprising lisdexamfetamine dimesylate.

### Accelerated Aging Test

The tablet composition was subjected to accelerated stability condition, such as elevated temperature of 40 degree C and humidity of 75% RH exposure to simulate long-term environmental exposure. The performance of the tablet comprising lisdexamfetamine dimesylate is evaluated before and after aging to assess its resistance to degradation over time.

In TABLE 17, the results for the accelerated aging test performed on the tablet composition comprising lisdexamfetamine dimesylate is depicted.

**TABLE 17**

| **Category / Properties** | **Analysis** |
|---|---|
| Initial Hardness | Ranges from 30 N for lower strength (10 mg) and 70 N for higher strength (70 mg) |
| Hardness on keeping the tablets under room temperature for 3 months | Ranges from 30 N for lower strength (10 mg) and 70 N for higher strength (70 mg) |

The result reveals no change in the hardness of the tablet composition comprising lisdexamfetamine dimesylate. No drop or change in the hardness of the tablet confirms that the material's mechanical properties and its ability to withstand stress and deformation without exhibiting signs of deliquescence.

### Chemical Resistance Test / Compatibility Test

The tablet composition was exposed to a range of chemical substances commonly used in DT formulation to assess the tablet's resistance to chemical attack, including changes in appearance, physical properties, or structural integrity.

For conducting the compatibility study, the tablets are exposed at elevated temperature of 40 degree C and humidity of 75% RH exposure in open and closed conditions and at temperature of 25 degree C and humidity of 75% RH exposure in open and closed conditions.

The result reveals positive interpretations indicating a high level of compatibility between the tablet composition comprising lisdexamfetamine dimesylate and chemical substances commonly used in DT formulation. The positive results from the compatibility study provide strong evidence supporting the suitability of excipients materials for their intended application.

### Moisture Absorption Test

In order to determine the ability of the tablet composition comprising lisdexamfetamine dimesylate to resist moisture absorption by subjecting it to controlled humidity or water immersion tests. The amount of moisture absorbed by the tablet is measured and its impact on dimensional stability, strength, and other relevant properties are assessed.

The tablet composition comprising lisdexamfetamine dimesylate by using KF analyser at different storage conditions temperature of 40 °C / Relative humidity 75%, temperature of 30°C / Relative humidity 75% temperature of 30 °C / Relative humidity 65% and temperature of 25 °C / Relative humidity 60%.

The results reveal that the absorption of moisture content of tablet composition comprising lisdexamfetamine dimesylate remains the same. Summarily, no significant increase in absorption of moisture level is shown by the tablet.

In TABLE 18, the results for the Moisture Absorption Test of tablet composition comprising lisdexamfetamine dimesylate for BATCH 1 is depicted.

**TABLE 18**

| **Parameters** | **Initial** | **1 month** | | | |
|---|---|---|---|---|---|
| | | **25°C/ 60%RH** | **30°C/ 65%RH** | **30°C/ 75%RH** | **40°C/ 75%RH** |
| **Average mass** | 702.2 mg | 700.8 mg | 704.1 mg | 704.6 mg | 703.6 mg |
| **Water Content** | 3.3% | 3.8% | 3.5% | 3.6% | 3.9% |
| **Disintegration Time** | 25 secs | 25 secs | 27 secs | 26 secs | 23 secs |
| **Dissolution (Paddle, 50 rpm, 900 ml 0.1N HCL)** | 100% | 101% | 100% | 102% | 101% |
| **Assay (By HPLC)** | 71.15 mg / tablet | 68.80 mg / tablet | 70.69 mg / tablet | 70.08 mg / tablet | 69.71 mg / tablet |
| | 101.6% | 98.3% | 101.0% | 100.1% | 99.6% |

In TABLE 19, the results for the Moisture Absorption Test of tablet composition comprising lisdexamfetamine dimesylate for BATCH 2 is depicted.

**TABLE 19**

| **Parameters** | **Initial** | **1 month** | | | |
|---|---|---|---|---|---|
| | | **25°C/ 60%RH** | **30°C/ 65%RH** | **30°C/ 75%RH** | **40°C/ 75%RH** |
| **Average mass** | 703.0 mg | 703.6 mg | 702.1 mg | 705.3 mg | 703.6 mg |
| **Water Content** | 3.4% | 3.8% | 3.4% | 3.9% | 3.4% |
| **Disintegration Time** | 23 secs | 22 secs | 23 secs | 31 secs | 28 secs |
| **Dissolution (Paddle, 50 rpm, 900 ml 0.1N HCL)** | 97% | 101% | 102% | 96% | 101% |
| **Assay (By HPLC)** | 70.87 mg / tablet | 69.64 mg / tablet | 69.62 mg / tablet | 70.32 mg / tablet | 69.05 mg / tablet |
| | 101.2% | 99.5% | 99.5% | 100.5% | 98.6% |

In TABLE 20, the results for the Moisture Absorption Test of tablet composition comprising lisdexamfetamine dimesylate for BATCH 3 is depicted.

**TABLE 20**

| **Parameters** | **Initial** | **1 month** | | | |
|---|---|---|---|---|---|
| | | **25°C/ 60%RH** | **30°C/ 65%RH** | **30°C/ 75%RH** | **40°C/ 75%RH** |
| **Average mass** | 703.3 mg | 704.0 mg | 703.2 mg | 702.3 mg | 700.8 mg |
| **Water Content** | 3.1% | 3.6% | 3.4% | 3.6% | 3.3% |
| **Disintegration Time** | 24 secs | 26 secs | 27 secs | 28 secs | 25 secs |
| **Dissolution (Paddle, 50 rpm, 900 ml 0.1N HCL)** | 98% | 99% | 102% | 102% | 96% |
| **Assay** | 70.71 mg / tablet | 69.40 mg / tablet | 70.66 mg / tablet | 70.06 mg / tablet | 69.20 mg / tablet |
| **(By HPLC)** | 101.0% | 99.1 % | 100.9% | 100.1% | 98.9% |

The stability report for BATCH 1, BATCH 2, and BATCH 3 emphasizes on non-deliquescent nature of the lisdexamfetamine dimesylate dispersible composition. From the stability data (shown in TABLE 18, TABLE 19 and TABLE 20), it can be interpreted that there is no significant increase in water content at mentioned storage conditions. Hence, in other words it can be concluded that lisdexamfetamine dimesylate composition for dispersible tablet is stable.

### Disintegration and fineness of dispersion test

As mentioned in, Dispersible tablets disintegrate within 3 min, using water R at 15-25 °C as the liquid medium.

European Pharmacopoeia further directs to place 2 tablets in 100 mL of water R and stir until completely dispersed. A smooth dispersion is produced, which passes through a sieve screen with a nominal mesh aperture of 710 µm.

On performing the tests for disintegration and fineness of dispersion for on the lisdexamfetamine dimesylate dispersible tablet prepared on European Pharmacopoeia following the method for preparation disclosed in the present disclosure, the test reveals that the disintegration time and the fineness of the dispersion complies with the accepted criteria as mentioned in the European Pharmacopoeia.

In TABLE 21, the results for tests performed for disintegration and fineness of dispersion for the dispersible tablets are depicted.

**TABLE 21**

| **Product** | **Lisdexamfetamine Dimesylate Dispersible Tablets** | | | | | | | |
|---|---|---|---|---|---|---|---|---|
| **Test Parameter** | **Acceptance Criteria** | **Strength** | | | | | | |
| | | **10 mg** | **20 mg** | **30 mg** | **40 mg** | **50 mg** | **60 mg** | **70 mg** |
| **Disintegra tion Time** | Not more than 3 minutes using water at 15°C-25°C, without disc. | 58 seconds | 35 seconds | 30 Seconds | 45 seconds | 48 seconds | 48 seconds | 46 seconds |
| **Fineness of Dispersion** | A smooth dispersion should produce which passes through a sieve screen with a nominal mesh aperture of 710 µₗm | Complies | Complies | Complies | Complies | Complies | Complies | Complies |

The present disclosure provides a method for preparation of lisdexamfetamine dimesylate dispersible tablet and the lisdexamfetamine dimesylate dispersible tablet prepared thereby. The lisdexamfetamine dimesylate dispersible tablet developed by the inventor is a dispersible tablet in multiple dosage amount which can be consumed with ease, which gets rapidly dissolved and have the characteristics of dissolving or melting or disintegrating in only a few seconds and providing desired therapeutic outcome. The present disclosure provides a method for preparation of dispersible tablet which enhances the disintegration; dissolution, and free flowability of the components of the granule in the dosage form by its physio-chemical parameters adopted during the method.

The lisdexamfetamine dimesylate dispersible tablet addresses the deliquescence problem of lisdexamfetamine dimesylate. The lisdexamfetamine dimesylate dispersible tablet disintegrates fast and permits rapid release of the drug from the granules and allows rapid absorption in the body after administration. Further, the lisdexamfetamine dimesylate dispersible tablet is stable, palatable, and disintegrates fast. Moreover, the method for preparation of lisdexamfetamine dimesylate tablet involves lesser process time and production cost. Furthermore, the method for preparation has controlled humidity to minimize exposure of the deliquescent material to moisture during the production of dispersible tablet.

The lisdexamfetamine dimesylate dispersible tablets are quickly disintegrable or soluble and the tablet in dispersible form on consumption lets the drug be absorbed in the gastrointestinal regions.

The present disclosure provides a synergistic combination of the API with the excipients in specific ratio to address the deliquescent nature of lisdexamfetamine dimesylate. The synergistic combination enhances the disintegration time of the dispersible tablet. The present disclosure further imparts improved palatability to the tablet formulation of lisdexamfetamine dimesylate. The present disclosure provides a humidity-controlled method of preparation wherein the tablet formulation is manufactured in an environment with controlled humidity to minimize exposure of the deliquescent material to moisture during the production process. Particularly, the manufacturing process employs direct compression techniques during the manufacturing process in controlled humidity level of NMT 40% to reduce exposure to moisture.

The present disclosure provides a method for preparation of lisdexamfetamine dimesylate composition for dispersible tablet which overcomes the drawbacks of solid compositions like capsules and chewable tablets. The synergistic combination of the API with the excipients in specific ratio is formulated to address the deliquescent nature of lisdexamfetamine dimesylate and provide a stable lisdexamfetamine dimesylate dispersible composition. The dissolution of the lisdexamfetamine dispersible tablet composition is not less than 75% in 15 minutes.

The foregoing descriptions of specific embodiments of the present disclosure have been presented for purposes of illustration and description. They are not intended to be exhaustive or to limit the present disclosure to the precise forms disclosed, and obviously many modifications and variations are possible in light of the above teaching. The embodiments were chosen and described in order to best explain the principles of the present disclosure and its practical application, to thereby enable others skilled in the art to best utilize the present disclosure and various embodiments with various modifications as are suited to the particular use contemplated. (It is understood that various omissions and substitutions of equivalents are contemplated as circumstance may suggest or render expedient, but such are intended to cover the application or implementation without departing from the spirit or scope of the claims of the present disclosure).

## Claims

1. A method for preparation of a lisdexamfetamine dimesylate dispersible tablet, the method comprising:
dry mixing lisdexamfetamine dimesylate, a diluent, a binder and an anti-static agent to form a dry mix blend;
intermediate granular blending of a filler, a cushioning agent, a disintegrating agent, and a sweetener to form an intermediate granular blend;
adding and blending the dry mix blend and the intermediate granular blend to form an intermediate drug blend;
lubricating the intermediate drug blend with a lubricant to form ready to compress drug granules; and
compressing the ready to compress drug granules to form a lisdexamfetamine dimesylate dispersible tablet.

2. The method as claimed in claim 1, wherein the lisdexamfetamine dimesylate is present in an amount of about 8 to about 12 percent by weight.

3. The method as claimed in claim 1, wherein in the dry mixing step:
the diluent is mannitol granular 200SD, and the diluent is present in an amount of about 18 to about 23 percent by weight;
the binder is partially pregelatinized maize starch, and the binder is present in an amount of about 10 to about 20 percent by weight; and
the anti-static agent is colloidal anhydrous silica, and the anti-static agent is present in an amount of about 1 to about 2 percent by weight.

4. The method as claimed in claim 1, wherein in the intermediate granular blending step:
the filler is mannitol granular 200SD, and the filler is present in an amount of about 28 to about 34 percent by weight;
the cushioning agent is microcrystalline cellulose, and the cushioning agent is present in an amount of about 8 to about 12 percent by weight;
the disintegrating agent is crospovidone, and the disintegrating agent is present in an amount of about 6 to about 10 percent by weight; and
the sweetener is sucralose, and the sweetener is present in an amount of about 1 to about 4 percent by weight.

5. The method as claimed in claim 1, wherein in the lubrication step, the lubricant is magnesium stearate, and the lubricant is present in an amount of about 1 to about 2 percent by weight.

6. The method as claimed in claim 1, wherein the dosage amount of the lisdexamfetamine dimesylate dispersible tablet is 10 mg per tablet; 20 mg per tablet; 30 mg per tablet; 40 mg per tablet; 50 mg per tablet; 60 mg per tablet; and 70 mg per tablet.

7. The method as claimed in claim 1, wherein the dissolution is not less than 75% in 15 minutes.

8. The method as claimed in claim 1, wherein the friability is not more than 1.5%, the disintegration time is less than 3 minutes at 15°C to 25°C (without disc), and the hardness is from about 30 N to about 70 N.

9. The method as claimed in claim 1, wherein the step of compressing employs direct compression technique in controlled humidity level of NMT 40% to reduce exposure to moisture.

10. The method as claimed in claim 1, further comprising packaging of lisdexamfetamine dimesylate dispersible tablets in moisture resistant ALU peel off blister package; and/or moisture resistant ALU-ALU blister package.
